(19) Europäisches Patentamt
European Patent Office
Office européen des brevets

(11) **EP 3 031 385 A1**

(12) **EUROPEAN PATENT APPLICATION**
published in accordance with Art. 153(4) EPC

(43) Date of publication:
**15.06.2016 Bulletin 2016/24**

(51) Int Cl.:
*A61B 1/00* (2006.01)    *A61B 1/04* (2006.01)
*A61B 5/06* (2006.01)

(21) Application number: **14834950.9**

(22) Date of filing: **30.06.2014**

(86) International application number:
**PCT/JP2014/067351**

(87) International publication number:
**WO 2015/019745 (12.02.2015 Gazette 2015/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **06.08.2013   JP 2013163348**

(71) Applicant: **Olympus Corporation**
**Shibuya-ku**
**Tokyo 151-0072 (JP)**

(72) Inventors:
• **TOJO, Ryo**
 **Tokyo 151-0072 (JP)**
• **ITO, Takeshi**
 **Tokyo 151-0072 (JP)**

(74) Representative: **Winter, Brandl, Fürniss, Hübner, Röss, Kaiser, Polte - Partnerschaft mbB**
**Patent- und Rechtsanwaltskanzlei**
**Alois-Steinecker-Strasse 22**
**85354 Freising (DE)**

(54) **INSERTION SYSTEM AND METHOD FOR ADJUSTING SHAPE DETECTION CHARACTERISTICS OF SHAPE SENSOR**

(57)    An insertion system (1) includes an insertion device (100), a shape sensor (130), an insertion device holder (420) and an adjustment unit (230). The insertion device (100) includes an elongated flexible insertion portion (110). The shape sensor (130) detects a shape of the insertion portion (110). The insertion device holder (420) holds the insertion device (100) at one end of the insertion portion (110) so that the other end of the insertion portion (110) hangs. The adjustment unit (230) determines an adjustment value to adjust shape detection characteristics of the shape sensor (130) when the insertion device (100) is held by the insertion device holder (420).

Adjustment value determination processing

S101  Acquire holding state

S102  Insertion device is held by insertion device holder? — NO

YES

S103  Compare current light receiving amount with light receiving amount in initial state in which insertion portion is straight

S104  Adjust relation between current light receiving amount and light receiving amount in initial state in which insertion portion is straight

End

F I G. 7

EP 3 031 385 A1

## Description

Technical Field

The present invention relates to an insertion system and a method of adjusting shape detection characteristics of a shape sensor.

Background Art

In general, an insertion system having an elongated portion which is provided with a camera at the distal end is known as a system for photographing a space having a narrow entrance. Such an insertion system includes various endoscopes. There is known an insertion system in which an elongated portion provided with a camera at the distal end is flexible. Such an insertion system may be provided with a mechanism for acquiring the shape of the flexible portion. For example, Jpn. Pat. Appln. KOKAI Publication No. 2003-052614 discloses an art related to a fiber sensor for acquiring the shape of a flexible portion of an endoscope.

The following endoscope is disclosed in Jpn. Pat. Appln. KOKAI Publication No. 2003-052614. The endoscope is provided with flexible bending detection optical fibers. The bending detection optical fibers have bending detectors, and the transmission amount of light changes in accordance with the bending angles of the bending detectors. The bending detection optical fibers are attached to a flexible belt-shaped member so that the detectors are arranged, and inserted and disposed in the endoscope over the entire length. The detection optical fibers having such detectors function as a fiber sensor which is a shape sensor. That is, the curving state of the belt-shaped member in the part where each of the bending detectors is located is detected on the basis of the light transmission amount of each of the detection optical fibers. This curving state is displayed on a monitor screen as the curving state of the endoscope.

Summary of Invention

Shape sensors for shape detection including the fiber sensor disclosed in Jpn. Pat. Appln. KOKAI Publication No. 2003-052614 may change in output characteristics with time. To maintain high detection precision, it is necessary to calibrate the shape sensor before use.

Accordingly, an object of the present invention is to provide an insertion system in which a shape sensor is easily calibrated and a method of easily adjusting shape detection characteristics of the shape sensor.

To achieve the above described object, according to an aspect of the invention, an insertion system includes an insertion device which comprises an elongated flexible insertion portion; a shape sensor which detects a shape of the insertion portion; an insertion device holder which holds the insertion device at one end of the insertion portion so that the other end of the insertion portion hangs; and an adjustment unit which determines an adjustment value to adjust shape detection characteristics of the shape sensor when the insertion device is held by the insertion device holder.

According to an aspect of the invention, a method of adjusting shape detection characteristics of a shape sensor of an insertion system, the insertion system comprising an insertion device which includes an elongated flexible insertion portion and the shape sensor which detects a shape of the insertion portion, includes detecting whether the insertion device is held at one end of the insertion portion so that the other end of the insertion portion hangs; and determining an adjustment value to adjust the shape detection characteristics of the shape sensor when the insertion device is held.

According to the present invention, it is possible to provide an insertion system in which a shape sensor is easily calibrated and a method of easily adjusting shape detection characteristics of the shape sensor.

Brief Description of Drawings

FIG. 1 is a diagram showing an overview of the appearance of an example of an insertion system according to a first embodiment;
FIG. 2 is a block diagram showing an overview of a configuration example of the insertion system according to the first embodiment;
FIG. 3A is a diagram illustrating a fiber sensor;
FIG. 3B is a diagram illustrating the fiber sensor;
FIG. 3C is a diagram illustrating the fiber sensor;
FIG. 4 is a diagram showing an overview of a configuration example of an insertion portion;
FIG. 5 is a diagram illustrating an example of a holding state detector according to the first embodiment;
FIG. 6 is a graph showing an example of the relation between the curvature (curving amount) of the insertion portion and the light receiving amount;
FIG. 7 is a flowchart showing an example of adjustment value determination processing according to the first embodiment;
FIG. 8 is a diagram illustrating an example of the holding state detector according to a first modification of the first embodiment;
FIG. 9 is a diagram illustrating an example of the holding state detector according to a second modification of the first embodiment;
FIG. 10 is a block diagram showing an overview of a configuration example of the insertion system according to a third modification of the first embodiment;
FIG. 11 is a flowchart showing an example of adjustment value determination processing according to a fourth modification of the first embodiment;
FIG. 12 is a diagram showing an overview of a configuration example of the insertion system according

FIG. 13 is a flowchart showing an example of adjustment value determination processing according to the second embodiment;

FIG. 14 is a diagram showing an overview of a configuration example of the insertion system according to a first modification of the second embodiment;

FIG. 15 is a flowchart showing an example of adjustment value determination processing according to a second modification of the second embodiment;

FIG. 16 is a diagram showing an overview of a configuration example of the insertion system according to a first example of a third embodiment;

FIG. 17 is a diagram showing an overview of a configuration example of the insertion system according to a second example of the third embodiment;

FIG. 18A is a diagram showing that a state determination pattern is imaged in the center;

FIG. 18B is a diagram showing that the state determination pattern is imaged out of position;

FIG. 18C is a diagram showing that no state determination pattern is imaged; and

FIG. 19 is a diagram showing an overview of a configuration example of the insertion system according to a third example of the third embodiment.

Description of Embodiments

[First Embodiment]

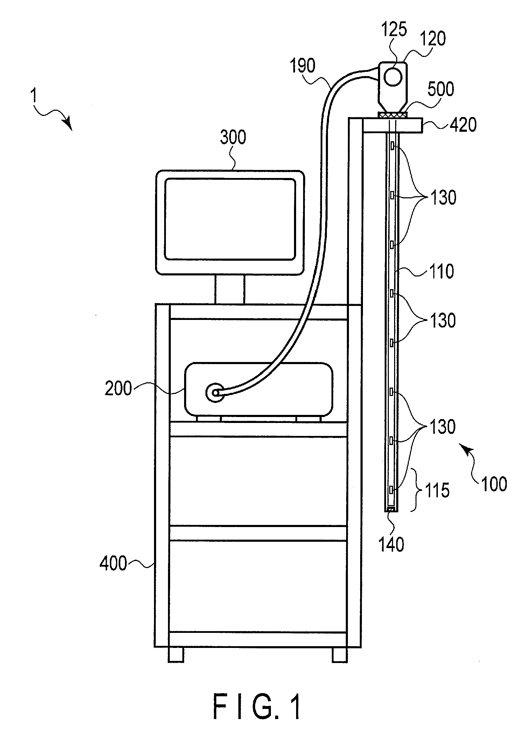

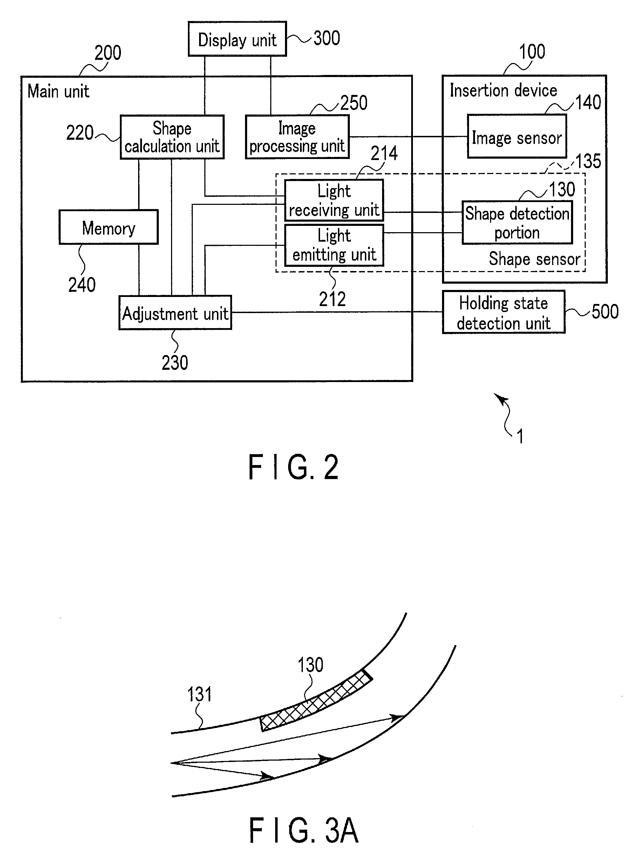

A first embodiment is described with reference to the drawings. The present embodiment relates to a medical endoscope as an example of an insertion system. An overview of a configuration example of an insertion system 1 according to the present embodiment is shown in FIG. 1 and FIG. 2. FIG. 1 is an image diagram showing an overview of the appearance of the insertion system 1. FIG. 2 is a block diagram showing an overview of a configuration example of the insertion system 1.

As shown in FIG. 1 and FIG. 2, the insertion system 1 comprises an insertion device 100, a main unit 200, and a display unit 300. The insertion device 100 is, for example, an endoscope. The main unit 200 is connected to the insertion device 100, and controls the insertion device 100 and performs various calculations. The display unit 300 is a general display connected to the main unit 200, and displays images obtained by the insertion device 100, the shape of the insertion device 100, and various control parameters.

The insertion device 100 is inserted into an insertion target to image the inside of the insertion target. An obtained image is displayed on the display unit 300. The insertion system 1 has a mechanism which detects the current shape of the insertion device 100. The detected current shape of the insertion device 100 is displayed on the display unit 300.

As shown in FIG. 1, the main unit 200 and the display unit 300 are put on a rack 400. When not in use, the insertion device 100 is hung on an insertion device holder 420 which is a hanger provided in the rack 400. The insertion device holder 420 is provided with a holding state detection unit 500 which detects whether the insertion device 100 is disposed.

The insertion device 100 includes an elongated flexible insertion portion 110, and an operation portion 120 provided on the proximal side of the insertion portion 110. The insertion portion 110 is inserted into the insertion target. A bending section 115 which actively bends is provided in the vicinity of the distal end of the insertion portion 110. The operation portion 120 is a portion to be grasped by a user, and has various operational parts for the user to perform operations associated with the insertion portion 110. For example, the operation portion 120 is provided with an operation knob 125 for changing the bending state of the bending section 115. The operation portion 120 and the main unit 200 are connected to each other by a cable 190.

The insertion portion 110 is provided with a shape detection portion 130 which detects the shape of the insertion portion 110. An image sensor 140 is provided at the distal end of the insertion device 100. An image of the inside of the insertion target is acquired by the image sensor 140. An image signal obtained by use of the image sensor 140 is sent to the main unit 200 via the cable 190. An unshown light emitting portion which emits light for illumination is provided at the distal end of the insertion portion 110. The illumination light guided in the cable 190 and the insertion portion 110 from the main unit 200 via optical fibers is emitted from the light emitting portion.

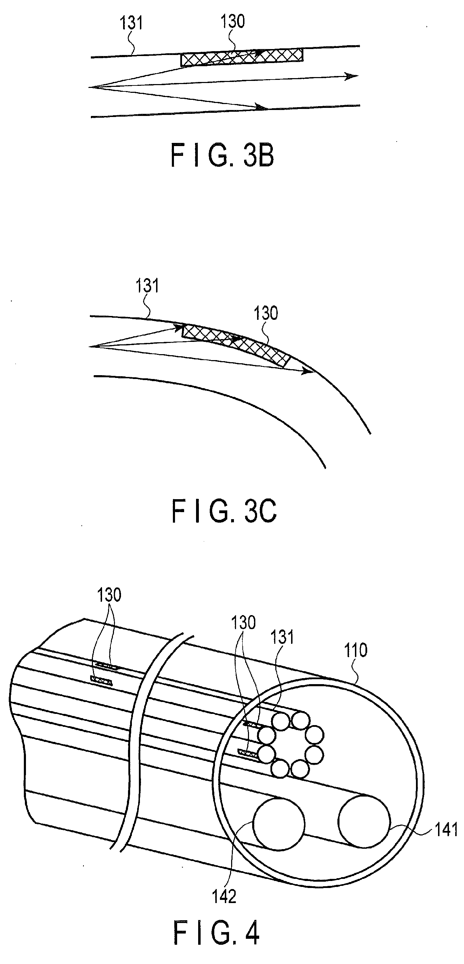

The shape detection portion 130 is briefly described. For example, a fiber sensor is used as a shape sensor including the shape detection portion 130. An example of the fiber sensor is described with reference to FIG. 3A, FIG. 3B, FIG. 3C, and FIG. 4. The fiber sensor comprises an optical fiber 131. As shown in FIG. 2, the main unit 200 is provided with a light emitting unit 212 which emits light guided by the optical fiber, and a light receiving unit 214 which receives light guided by the optical fiber.

The operation principle of the fiber sensor is described. The optical fiber 131 is provided with the shape detection portion 130. In the shape detection portion 130, cladding of the optical fiber 131 is removed so that its core is exposed, and this part is coated with a light absorbing material. As a result, the intensity of light guided by the optical fiber 131 changes depending on the state of the curving of the optical fiber 131. The shape detection portion 130 shown in FIG. 1 schematically represents the position of the shape detection portion 130 provided in the optical fiber 131.

For example, when the optical fiber 131 is curved so that the shape detection portion 130 comes inside as shown in FIG. 3A, the light transmission rate by the optical fiber 131 is higher. In contrast, when the optical fiber 131 is curved so that the shape detection portion 130 comes outside as shown in FIG. 3C, the light transmission rate by the optical fiber 131 is lower. When the optical fiber 131 is not curved as shown in FIG. 3B, the light transmission rate by the optical fiber 131 is lower than that in the case shown in FIG. 3A and higher than that in the case shown in FIG. 3C. Such an optical fiber 131 is inserted through the insertion portion 110. Light emitted from the light emitting unit 212 provided in the main unit 200 enters the optical fiber 131, passes through the shape detection portion 130, and is then again guided to the main unit 200, and is detected by the light receiving unit 214. The light receiving unit 214 measures the intensity of the light guided by the optical fiber 131. A curving amount of the insertion portion 110 in the region where the shape detection portion 130 is provided is calculated on the basis of the measured intensity of the received light.

As shown in FIG. 4, a bundle of optical fibers 131 are disposed in the insertion portion 110. To detect curving amounts in two directions (e.g., an X-axis direction and a Y-axis direction) that intersect at right angles in each part of the insertion portion 110, the optical fiber 131 in which the shape detection portion 130 is provided in one direction (e.g., the X-axis direction) and the optical fiber 131 in which the shape detection portion 130 is provided in a direction (e.g., the Y-axis direction) that intersects at right angles with the one direction are provided in pairs in the insertion portion 110. Moreover, the optical fibers 131 in which the shape detection portions 130 are provided at different positions in the longitudinal axis direction of the insertion portion 110 are provided in the insertion portion 110. In FIG. 4, an illumination light optical fiber 142 which transmits the illumination light emitted from the distal end of the insertion portion 110, and a wiring line 141 for the image sensor 140 are drawn in addition to the optical fibers 131.

Although one shape detection portion 130 is provided in one optical fiber 131 in the example shown here, more than one shape detection portion 130 may be provided in one optical fiber. For example, each of the shape detection portions 130 may be coated with a light absorbing material having different wavelength characteristics so that the light intensity of different wavelengths change on the basis of the curving amount of each of the shape detection portions 130.

Thus, the shape detection portion 130, the optical fiber 131, the light emitting unit 212, and the light receiving unit 214, for example, form a shape sensor 135 as a whole.

Back to FIG. 2, the explanation of the configuration of the insertion system 1 is carried on. As shown in FIG. 2, the main unit 200 is provided with a shape calculation unit 220, an adjustment unit 230, a memory 240, and an image processing unit 250. The shape calculation unit 220 acquires a light receiving amount associated with intensity of the light associated with the shape detection portion 130 from the light receiving unit 214. On the basis of this light receiving amount, the shape calculation unit 220 calculates the shape of the insertion portion 110. The shape calculation unit 220 displays the calculated shape of the insertion portion 110 on the display unit 300.

A relational expression between a change of the light transmission amount Δl of the optical fiber of the shape sensor 135 and a curving amount φ of the shape detection portion 130 is given, for example, as in Equation (1):

$$\phi = f(\Delta l) \quad (1).$$

The shape calculation unit 220 calculates the curving amount φ of each of the shape detection portions 130 on the basis of Equation (1) and the change of the light transmission amount Δl. Moreover, the shape calculation unit 220 calculates the shape of the insertion portion from the curving amount φ of each of the shape detection portions 130 and known information regarding the distance between the shape detection portions 130. The shape calculation unit 220 does not need to directly calculate by use of Equation (1). That is, a conversion table corresponding to Equation (1) is prepared, and the shape calculation unit 220 may calculate the curving amount φ on the basis of this conversion table.

The adjustment unit 230 is connected to the light emitting unit 212, the light receiving unit 214, the shape calculation unit 220, and the holding state detection unit 500. The adjustment unit 230 detects the state regarding the shape sensor 135, determines an adjustment value, and adjusts the characteristics regarding the shape sensor 135 on the basis of the adjustment value. The shape calculation unit 220 and the adjustment unit 230 are connected to the memory 240. The shape calculation unit 220 and the adjustment unit 230 perform various calculations by use of information stored in the memory 240 as appropriate. For example, Equation (1) and the corresponding conversion table are stored in the memory 240.

The image processing unit 250 is connected to the image sensor 140 of the insertion device 100. The image processing unit 250 creates a display image on the basis of image data obtained by the image sensor 140, and displays the display image on the display unit 300.

The insertion device holder 420 and the holding state detection unit 500 are described. The insertion device holder 420 holds the operation portion 120 of the insertion device 100 or the part located in the vicinity of the operation portion 120. Here, the part located in the vicinity of the operation portion 120 means, for example, a range of the operation portion 120 and a connection cable in the vicinity of the operation portion 120. The insertion device holder 420 may hold the insertion portion 110. However, when, for example, a human body is assumed as the insertion target, the insertion portion 110 needs to be kept clean. Therefore, the insertion device holder 420 preferably holds parts other than the insertion portion 110.

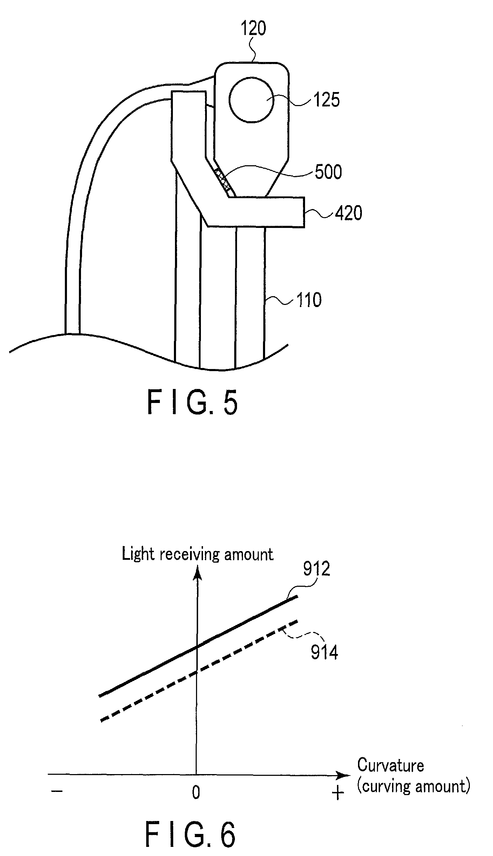

The holding state detection unit 500 is described with reference to FIG. 5. The holding state detection unit 500 includes, for example, a mechanical switch provided in the insertion device holder 420. The holding state detection unit 500 is located to intervene between the insertion device holder 420 and the operation portion 120 when the insertion device holder 420 holds the operation portion 120 of the insertion device 100. When the operation portion 120 is held by the insertion device holder 420, the switch of the holding state detection unit 500 is pushed and turned on by the weight of the insertion device 100. The holding state detection unit 500 outputs, to the adjustment unit 230, information regarding whether the insertion device 100 is held by the insertion device holder 420.

The operation of the insertion system 1 according to the present embodiment is described. The insertion system 1 has a function of making an adjustment regarding the detection of the shape of the insertion portion 110 using the shape detection portion 130. The output of the shape detection portion 130 using the optical fiber 131 deteriorates with time. That is, in the long term, a contact state of an optical system regarding the shape detection portion 130 changes or an incorporation state of the shape detection portion 130 in the insertion portion 110 changes, thus the characteristics of the intensity of light received by the light receiving unit 214 may change.

An example of the relation between the curvature (curving amount) of the insertion portion 110 and the light receiving amount is schematically shown in FIG. 6. For example, the relation between the curvature and the light receiving amount is initially a relation indicated by a solid line 912. In the shape detection portion 130 having such characteristics, if light guiding efficiency decreases due to dust which has come into optical contact with the optical system of the shape detection portion 130, the relation between the curvature and the light receiving amount changes as indicated by a dashed line 914. For example, when Equation (1) is based on the initial relation indicated by the solid line 912, the curving amount is not precisely calculated if this relation changes.

Thus, the insertion system 1 according to the present embodiment has a mechanism which calibrates the change of the output value regarding the shape detection portion 130 over time to perform a correct calculation of the curving amount.

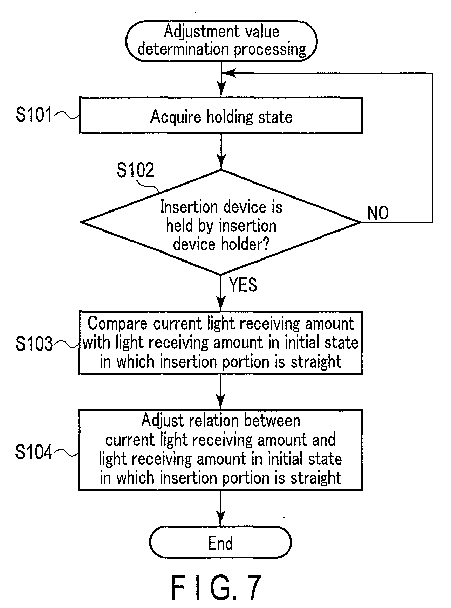

Adjustment value determination processing regarding the adjustment unit 230 of the insertion system 1 is described with reference to a flowchart shown in FIG. 7. In step S101, the adjustment unit 230 acquires information regarding the holding state from the holding state detection unit 500. In step S102, the adjustment unit 230 determines whether the insertion device 100 is held by the insertion device holder 420. When it is determined that the insertion device 100 is not held by the insertion device holder 420, the processing returns to step S101. In contrast, when it is determined that the insertion device 100 is held by the insertion device holder 420, the processing proceeds to step S103.

When the insertion device 100 is held by the insertion device holder 420, the insertion portion 110 is hanging straight. The adjustment unit 230 adjusts by the following processing so that the output regarding the shape detection portion 130 in this instance may be an output adapted to the initial state, that is, the relation in, for example, Equation (1).

That is, in step S103, the adjustment unit 230 temporarily turns on the light emitting unit 212 to obtain the light receiving amount, and compares the current light receiving amount output from the light receiving unit 214 with the light receiving amount in the initial state in which the insertion portion 110 is straight. In step S104, the adjustment unit 230 determines and outputs an adjustment value for adjusting the relation between the current light receiving amount and the light receiving amount in the initial state in which the insertion portion 110 is straight. When the adjustment value is determined, this fact may be displayed on the display unit 300. After step S104, the adjustment value determination processing ends.

It is possible to conceive several modes of methods of adjusting the relation between the current light receiving amount and the light receiving amount in the initial state in which the insertion portion 110 is straight. This adjustment can be made by, for example, the change of the light emitting intensity of the light emitting unit 212. That is, the light emitting intensity of the light emitting unit 212 is adjusted so that the current light receiving amount may be equal to the light receiving amount in the initial state in which the insertion portion 110 is straight. For example, when the current light receiving amount is lower than the light receiving amount in the initial state, the light emitting intensity is increased so that the current light receiving amount may be equal to the light receiving amount in the initial state. In this case, the adjustment value is output to the light emitting unit 212, and the light emitting unit 212 uses this value to adjust the light emitting intensity.

This adjustment can be made by, for example, the exposure time of the light receiving unit 214 or the change of a gain. That is, the exposure time of the light receiving unit 214 or the gain is adjusted so that an output value attributed to the current light receiving amount may be equal to an output value attributed to the light receiving amount in the initial state in which the insertion portion 110 is straight. In this case, the adjustment value is output to the light receiving unit 214, and the light receiving unit 214 uses this value to adjust the light receiving amount.

This adjustment can also be made by, for example, the change of Equation (1) or the conversion table used in the shape calculation unit 220. In this case, the adjustment value is output to the shape calculation unit 220, and the shape calculation unit 220 uses this value to perform a shape calculation. A combination of these adjustments may be used.

After that, when the insertion device 100 is used, the shape calculation unit 220 calculates the shape of the insertion portion 110 on the basis of a value regarding the shape sensor 135 adjusted by the adjustment unit 230. The shape calculation unit 220 displays the calculated shape of the insertion portion 110 on the display unit 300. The user operates the insertion device 100 while checking the shape of the insertion portion 110 displayed on the display unit 300, and then, for example, observes the inside of the insertion target.

According to the present embodiment, information regarding the calculation of the shape of the insertion portion 110 by the shape calculation unit 220 is adjusted by the adjustment unit 230, so that the shape calculation unit 220 can precisely calculate the shape of the insertion portion 110 regardless of the change over time. This adjustment value determination processing by the adjustment unit 230 is performed when the insertion device 100 is disposed in the insertion device holder 420 without being used. Therefore, the user does not need to do special work for this adjustment. Thus, according to the present embodiment, the adjustment is simply and easily made without any burden on the user. Moreover, whether the insertion device 100 is disposed in the insertion device holder 420 is determined on the basis of the signal output from the holding state detection unit 500. That is, this adjustment is automatically started, and the user is not troubled by the adjustment. The mechanical switch is used in the holding state detection unit 500 according to the present embodiment. According to the mechanical switch, whether the insertion device 100 is disposed in the insertion device holder 420 can be detected by a simple configuration.

As the present embodiment, the insertion system according to the medical endoscope has been described by way of example. However, the technique according to the present embodiment is not only applicable to the medical endoscope but also applicable to various insertion systems such as an industrial endoscope and a manipulator having an elongated shape.

Although the insertion device holder 420 holds the operation portion 120 in the example that has been shown, the insertion device holder 420 may hold the distal end of the insertion portion 110 so that the operation portion 120 hangs. However, it is preferable that the insertion device holder 420 holds the operation portion 120 as in the present embodiment for the reasons of insertion device strength, easiness of holding, and protection of the distal end of the insertion portion 110.

The fiber sensor that uses the optical fiber as the shape sensor 135 has been described in the example according to the present embodiment. However, the shape sensor 135 is not limited to the fiber sensor. The technique according to the present embodiment can be used in various sensors which change the output value with time.

[First Modification of First Embodiment]

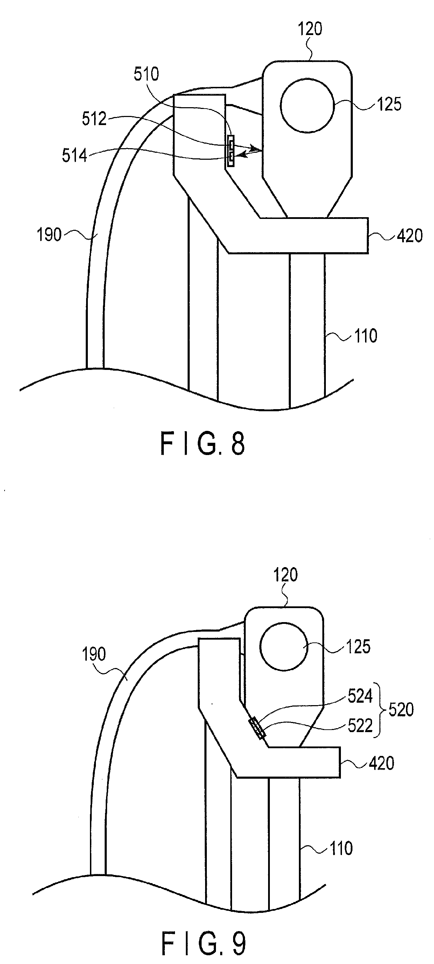

A first modification of the first embodiment is described. Here, the differences between the first modification and the first embodiment are described, and the same parts are denoted with the same reference signs and are not described. The present modification is different from the first embodiment in the configuration of the holding state detector. An overview of a configuration example of a holding state detector 510 according to the present modification is shown in FIG. 8.

The holding state detection unit 500 according to the first embodiment includes the mechanical switch. In contrast, the holding state detector 510 according to the present modification includes an optical switch as shown in FIG. 8. That is, the holding state detector 510 has a light emitting portion 512 and a light receiving portion 514. The light emitting portion 512 emits light.

When the operation portion 120 of the insertion device 100 is disposed in the insertion device holder 420, the light emitted from the light emitting portion 512 is applied to the operation portion 120 of the insertion device 100. This light is reflected in the operation portion 120. The light reflected in the operation portion 120 enters the light receiving portion 514. The light receiving portion 514 receives the reflected light which has arrived from the operation portion 120, and outputs a signal which indicates that the light has been received. In contrast, when the operation portion 120 is not disposed in the insertion device holder 420, the light emitted from the light emitting portion 512 is not received by the light receiving portion 514.

Thus, the holding state detector 510 according to the present modification detects whether the operation portion 120 of the insertion device 100 is disposed in the insertion device holder 420 by whether the reflected light of the light emitted from the light emitting portion 512 is received by the light receiving portion 514. The adjustment unit 230 acquires a signal output from the light receiving portion 514. The adjustment unit 230 determines on the basis of the acquired signal whether the operation portion 120 is disposed in the insertion device holder 420, and controls the start of an adjustment operation. The present modification is similar in other respects to the first embodiment.

According to the present modification, the detection can be performed in a non-contact manner in contrast to the mechanical switch according to the first embodiment. Thus, in the insertion device holder 420, the region where the holding state detection unit 500 can be disposed increases, and the degree of freedom of designing increases.

[Second Modification of First Embodiment]

A second modification of the first embodiment is described. Here, the differences between the second modification and the first embodiment are described, and the same parts are denoted with the same reference signs and are not described. An overview of a configuration example of a holding state detector 520 according to the present modification is shown in FIG. 9. The holding state detector 520 according to the present modification has an electric switch. That is, the insertion device holder 420 is provided with a first electrode 522, and the operation portion 120 is provided with a second electrode 524. The first electrode 522 and the second electrode 524 are provided at positions to come into contact with each other when the operation portion 120 is disposed in the insertion device holder 420. When the first electrode 522 and the second electrode 524 contact and thus conduct, the holding state detector 520 according to the present modification outputs a signal which indicates that the operation portion 120 is disposed in the insertion device holder 420. The present modification is similar in other respects to the first embodiment.

In the present modification, the holding state detector 520 detects by electric connection whether the operation portion 120 is disposed in the insertion device holder 420. According to the present modification, even if, for example, something other than the insertion device 100 is erroneously disposed in the insertion device holder 420, the holding state detector 520 does not detect anything other than the operation portion 120 provided with the second electrode 524. That is, in the holding state detector 520 according to the present modification, there is no concern for erroneous detection attributed to something other than the insertion device 100 that is disposed in the insertion device holder 420. According to the holding state detector 520 in the present modification, it is also possible to determine whether the operation portion 120 is properly disposed in the insertion device holder 420.

[Third Modification of First Embodiment]

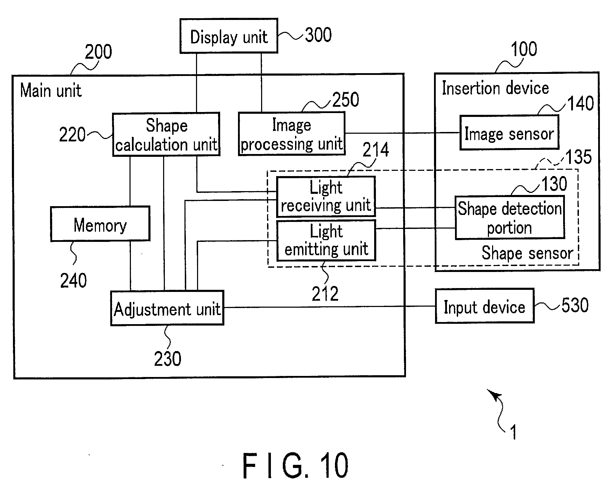

A third modification of the first embodiment is described. Here, the differences between the third modification and the first embodiment are described, and the same parts are denoted with the same reference signs and are not described. The insertion system 1 according to the present modification is provided with an input device 530 including, for example, a switch or a keyboard instead of the holding state detection unit 500, as shown in FIG. 10.

The user recognizes that the insertion device 100 is held by the insertion device holder 420, and operates the input device 530. In the present modification, the adjustment operation is started by the operation using the input device 530.

According to the present modification, the user can start the adjustment operation at any timing.

[Fourth Modification of First Embodiment]

A fourth modification of the first embodiment is described. Here, the differences between the fourth modification and the first embodiment are described, and the same parts are denoted with the same reference signs and are not described. The present modification is different from the first embodiment in the adjustment value determination processing.

When the insertion device 100 is held by the insertion device holder 420, the insertion portion 110 is not directly held and is merely hanging. Therefore, immediately after the insertion device 100 is held, the insertion portion 110 may be swinging without standing still due to the motion at the time of holding the insertion device 100 in the insertion device holder 420. Thus, according to the present embodiment, a time of waiting until the insertion portion 110 stands still is provided.

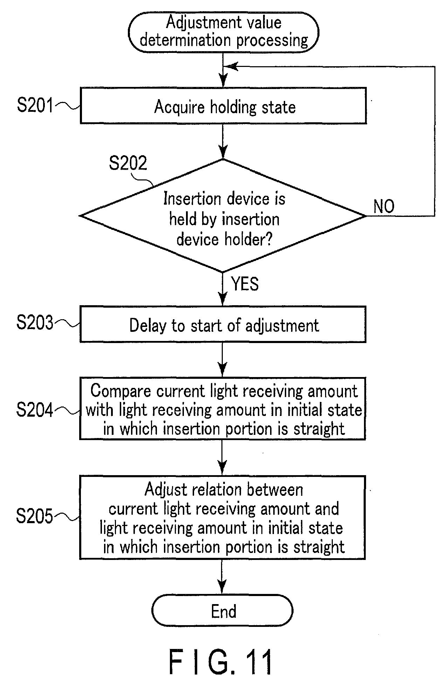

The adjustment value determination processing according to the present modification is described with reference to in FIG. 11. In step S201, the adjustment unit 230 acquires information regarding the holding state from the holding state detection unit 500. In step S202, the adjustment unit 230 determines whether the insertion device 100 is held by the insertion device holder 420. When it is determined that the insertion device 100 is not held by the insertion device holder 420, the processing returns to step S201. In contrast, when it is determined that the insertion device 100 is held by the insertion device holder 420, the processing proceeds to step S203.

In step S203, the adjustment unit 230 delays the processing to the start of the adjustment. That is, after it is detected that the insertion device 100 is held by the insertion device holder 420, the processing waits for a predicted time for the insertion portion 110 to come to a standstill. This wait time is suitably set, for example, to five seconds. Thus, the operation for the adjustment is performed after the insertion device 100 is held by the insertion device holder 420 and comes to a standstill.

In step S204, the adjustment unit 230 compares the current light receiving amount output from the light receiving unit 214 with the light receiving amount in the initial state in which the insertion portion 110 is straight. In step S205, the adjustment unit 230 determines and outputs an adjustment value for adjusting the relation between the current light receiving amount and the light receiving amount in the initial state in which the insertion portion 110 is straight. After step S205, the adjustment value determination processing ends.

According to the present modification, it is possible to prevent an adjustment value from being determined when the insertion portion 110 is moving immediately after the insertion portion 110 is disposed in the insertion device holder 420, that is, when the insertion portion 110 is not straight. As a result, it is possible to prevent the shape of the insertion portion 110 from being incorrectly calculated due to an error in the adjustment value.

[Second Embodiment]

A second embodiment is described. Here, the differences between the second embodiment and the first embodiment are described, and the same parts are denoted with the same reference signs and are not described. The insertion system 1 according to the present embodiment is provided with a mechanism which detects the bending state of the bending section 115.

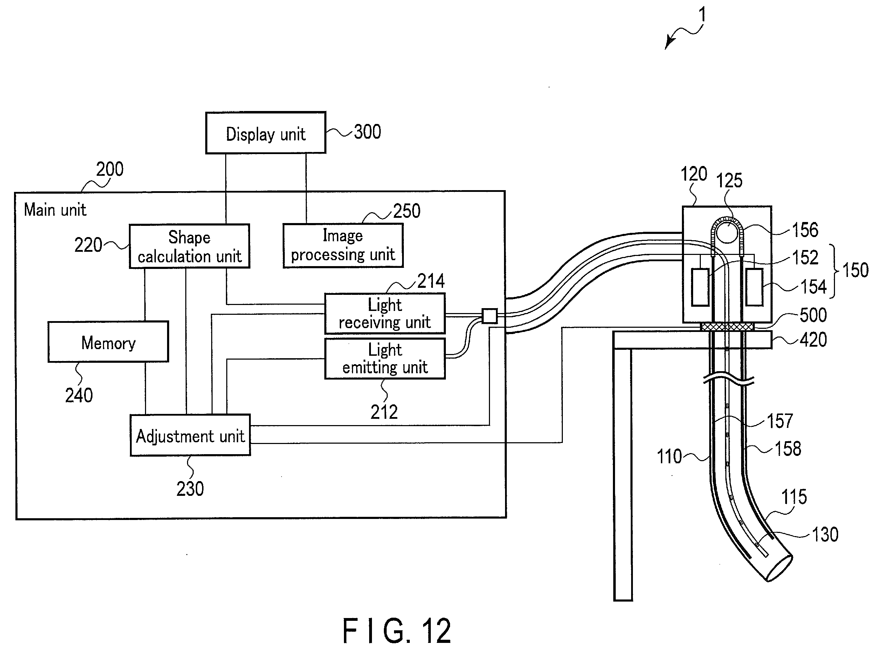

An overview of a configuration example of the insertion system 1 according to the present embodiment is shown in FIG. 12. As shown in this drawing, the insertion device 100 comprises a first operation wire 157 and a second operation wire 158 for bending the bending section 115. The first operation wire 157 and the second operation wire 158 are connected at one end by a coupling member 156 which is, for example, a chain. The coupling member 156 is wound around a pulley which interlocks with the operation knob 125 of the operation portion 120. The first operation wire 157 and the second operation wire 158 are connected at the other end to the bending section 115. When the operation knob 125 rotates, the coupling member 156 is displaced. In response to this displacement, the first operation wire 157 and the second operation wire 158 are displaced. As a result, the bending section 115 bends.

The operation portion 120 comprises a first displacement detection unit 152 which detects the displacement of the first operation wire 157, and a second displacement detection unit 154 which detects the displacement of the second operation wire 158. The first displacement detection unit 152 and the second displacement detection unit 154 form a bending state detection unit 150.

The first displacement detection unit 152 and the second displacement detection unit 154 are, for example, encoders. The first operation wire 157 and the second operation wire 158 are respectively provided with scales. The first displacement detection unit 152 and the second displacement detection unit 154 of a bending state detection unit 150 use the scales to detect the displacements of the first operation wire 157 and the second operation wire 158.

The bending state detection unit 150 outputs information regarding the displacements of the first operation wire 157 and the second operation wire 158 to the adjustment unit 230. The bending amount of the bending section 115 can be calculated on the basis of the displacements of the first operation wire 157 and the second operation wire 158.

Although the bending direction is only one direction (here, up/down) in the case described here, the same can be performed by further providing a third displacement detection unit and a fourth displacement detection unit in the case of bending in two directions, up/down and right/left. In the example shown here, the bending amount in one up/down direction is determined on the basis of the detection results in the first displacement detection unit 152 and the second displacement detection unit 154. The two detection units are used to reduce errors resulting from, for example, the slack of the wires. The two wires are coupled by the coupling member which is a chain, so that if an error is permitted, only one of the first displacement detection unit 152 and the second displacement detection unit 154 may be used.

The encoder has been shown as the bending state detection unit 150 here. However, the bending state detection unit 150 is not limited to this. For example, a potentiometer or a rotary encoder which measures the rotation amount of the operation knob 125 may be used as the bending state detection unit 150.

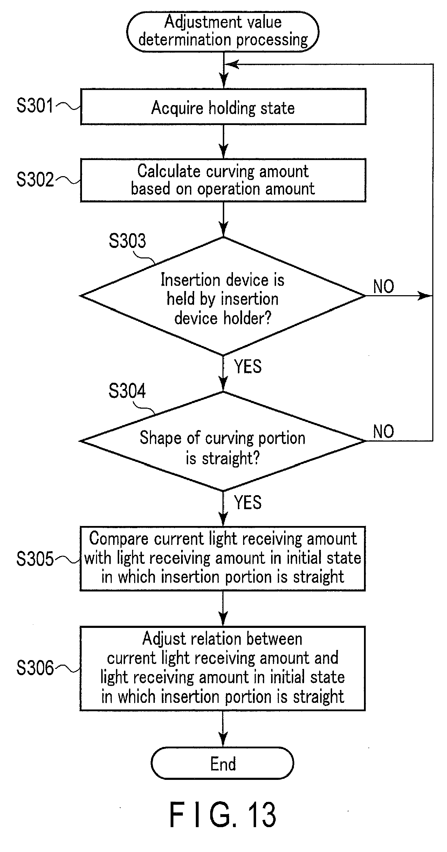

The adjustment unit 230 according to the present embodiment determines an adjustment value when the bending section 115 is, for example, straight. When the bending section 115 is not straight, no adjustment value is determined. The adjustment value determination processing by the adjustment unit 230 according to the present embodiment is described with reference to FIG. 13.

In step S301, the adjustment unit 230 acquires information regarding the holding state from the holding state detection unit 500. In step S302, the adjustment unit 230 acquires displacement amounts of the first operation wire 157 and the second operation wire 158 from the bending state detection unit 150, and calculates the bending amount of the bending section 115 on the basis of the displacement amounts.

In step S303, the adjustment unit 230 determines whether the insertion device 100 is held by the insertion device holder 420. When it is determined that the insertion device 100 is not held by the insertion device holder 420, the processing returns to step S301. In contrast, when it is determined that the insertion device 100 is held by the insertion device holder 420, the processing proceeds to step S304. In step S304, the adjustment unit 230 determines whether the shape of the bending section 115 is straight. When it is determined that the shape of the bending section 115 is not straight, the processing returns to step S301. In contrast, when it is determined that the shape of the bending section 115 is straight, the processing proceeds to step S305.

In step S305, the adjustment unit 230 compares the current light receiving amount output from the light receiving unit 214 with the light receiving amount in the initial state in which the insertion portion 110 is straight. In step S306, the adjustment unit 230 determines and outputs an adjustment value for adjusting the relation between the current light receiving amount and the light receiving amount in the initial state in which the insertion portion 110 is straight. After step S306, the adjustment value determination processing ends. Step S302 and step S303 may be in reverse order.

Thus, according to the present embodiment, an adjustment value is determined when the insertion device 100 is held by the insertion device holder 420 and the shape of the bending section 115 is straight. According to the present embodiment, errors in the adjustment value resulting from the determination of the adjustment value in states other than a predetermined state, for example, the state in which the bending section 115 is straight are inhibited.

Although an adjustment value is determined when the bending section 115 is straight in the example shown according to the present embodiment, an adjustment value may be determined, for example, when the bending section 115 is bent at 90°. In this case, the current output of the shape detection portion 130 can be adjusted to be equal to the output in the initial state in which the bending section 115 is bent at 90°. Similarly, the bending amount of the bending section 115 to determine an adjustment value may be set to any value.

Thus, for example, the first displacement detection unit 152 and the second displacement detection unit 154 function as insertion portion state detection units to acquire shape information regarding the shape of at least part of the insertion portion.

Although the insertion device 100 is held by the insertion device holder 420, the display unit 300 may be used to inform the user that an adjustment is impossible or to urge the user to bend the insertion portion 110 by a predetermined bending amount for starting an adjustment when the insertion portion 110 has not reached the predetermined bending amount.

[First Modification of Second Embodiment]

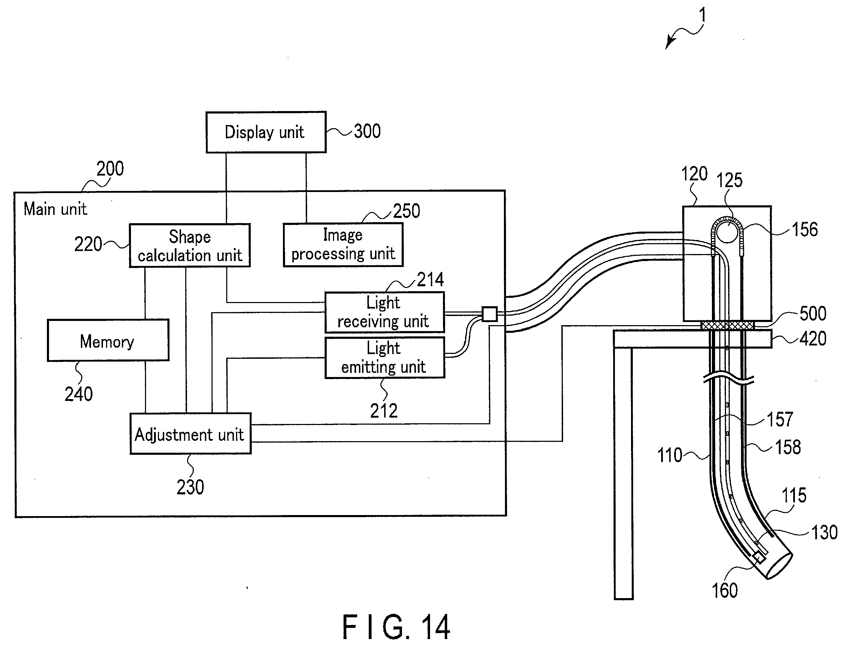

A first modification of the second embodiment is described. Here, the differences between the first modification and the second embodiment are described, and the same parts are denoted with the same reference signs and are not described. In the second embodiment, whether the bending section 115 is straight is determined on the basis of the output of the bending state detection unit 150 including the first displacement detection unit 152 and the second displacement detection unit 154. In contrast, in the present modification, a gravity sensor 160 is provided in the vicinity of the distal end of the insertion portion 110 as shown in FIG. 14. The adjustment unit 230 determines whether the bending section 115 is straight on the basis of the output of this gravity sensor 160.

That is, when the insertion portion 110 and the bending section 115 are straight, the distal end of the insertion portion 110 is vertically downward. When the gravity sensor 160 detects acceleration of gravity in the distal direction of the insertion portion 110, it is determined that the insertion portion 110 and the bending section 115 are straight. Thus, for example, the gravity sensor 160 functions as the insertion portion state detection unit to acquire shape information regarding the shape of at least part of the insertion portion.

Similarly, an unshown gravity sensor may be provided in the operation portion 120. On the basis of the output of the gravity sensor provided in the operation portion 120, it is possible to determine whether the operation portion 120 is held aslant or normally held to the insertion device holder 420. Thus, the state of the insertion portion 110 other than the bending section 115 can also be recognized.

[Second Modification of Second Embodiment]

A second modification of the second embodiment is described. Here, the differences between the second modification and the second embodiment are described, and the same parts are denoted with the same reference signs and are not described. In the present modification, an adjustment value is determined in accordance with the bending amount of the bending section 115 regardless of the shape of the bending section 115.

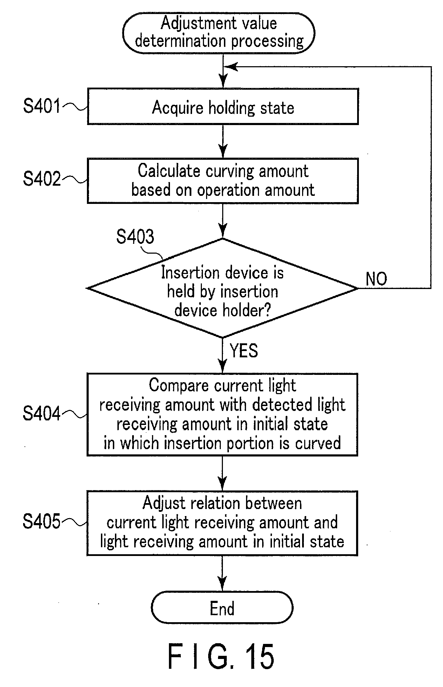

The adjustment value determination processing according to the present modification is described with reference to a flowchart shown in FIG. 15. In step S401, the adjustment unit 230 acquires information regarding the holding state from the holding state detection unit 500. In step S402, the adjustment unit 230 acquires displacement amounts of the first operation wire 157 and the second operation wire 158 from the bending state detection unit 150, and calculates the bending amount of the bending section 115 on the basis of the displacement amounts.

In step S403, the adjustment unit 230 determines whether the insertion device 100 is held by the insertion device holder 420. When it is determined that the insertion device 100 is not held by the insertion device holder 420, the processing returns to step S401. In contrast, when it is determined that the insertion device 100 is held by the insertion device holder 420, the processing proceeds to step S404.

In step S404, the adjustment unit 230 compares the current light receiving amount output from the light receiving unit 214 with the light receiving amount in the initial state in which the insertion portion 110 is in the same bending state as the current state. In step S405, the adjustment unit 230 determines and outputs an adjustment value for adjusting the relation between the current light receiving amount and the light receiving amount in the initial state in which the insertion portion 110 is in the same bending state as the current state. After step S405, the adjustment value determination processing ends. Step S402 and step S403 may be in reverse order.

According to the present embodiment, an adjustment value can be properly determined regardless of the state of the bending section 115.

[Third Embodiment]

A third embodiment is described. Here, the differences between the third embodiment and the first embodiment are described, and the same parts are denoted with the same reference signs and are not described. In the present embodiment, a holding state detection unit has a function of detecting that the insertion device 100 is held by the insertion device holder 420, and a function as an insertion portion state detection unit to acquire shape information regarding the shape of at least part of the insertion portion 110.

Although the holding state detection unit 500 according to the first embodiment, for example, the mechanical switch provided in the insertion device holder 420 is not provided in the example shown here, a mechanical switch provided in the insertion device holder 420 may be further provided.

(First Example)

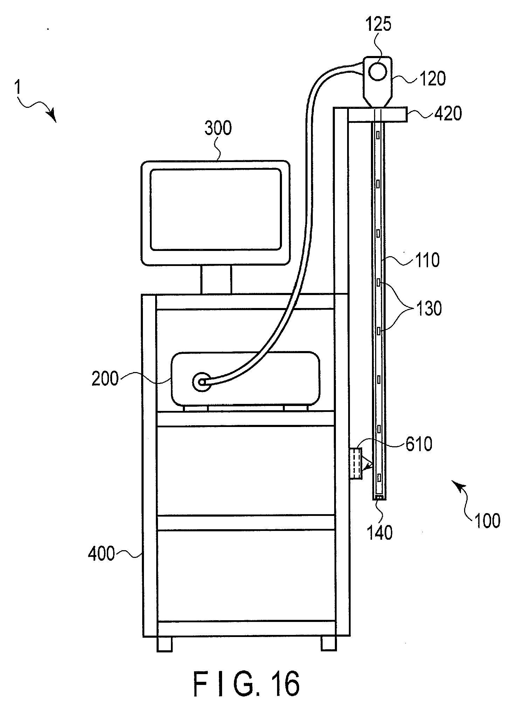

An example of an overview of the insertion system 1 according to the present embodiment is shown in FIG. 16. In this example, a proximity sensor 610 as a holding state detection unit is provided in the rack 400. This proximity sensor 610 is a sensor which detects that an object exists in the vicinity. The proximity sensor 610 is provided at a position to detect the distal end of the insertion portion 110 when the insertion device 100 is disposed in the insertion device holder 420 and the insertion portion 110 is hanging right down and is straight.

The adjustment unit 230 operates so that an adjustment value is determined when the proximity sensor 610 detects the distal end of the insertion portion 110. According to this example, it is possible to confirm, with the proximity sensor as the holding state detection unit alone, that the insertion device 100 is disposed in the insertion device holder 420 and that the insertion portion 110 is straight. As a result, determination of an abnormal adjustment value in states other than the state in which the insertion portion 110 is straight is prevented by a simple and easy configuration.

Although one proximity sensor 610 is provided in the example shown in FIG. 16, more than one proximity sensor may be disposed at different positions in the longitudinal direction of the insertion portion 110. When more than one proximity sensor is disposed, it is possible to more precisely determine whether the insertion portion 110 is straight. As a result, a more precise adjustment can be made by the adjustment unit 230.

(Second Example)

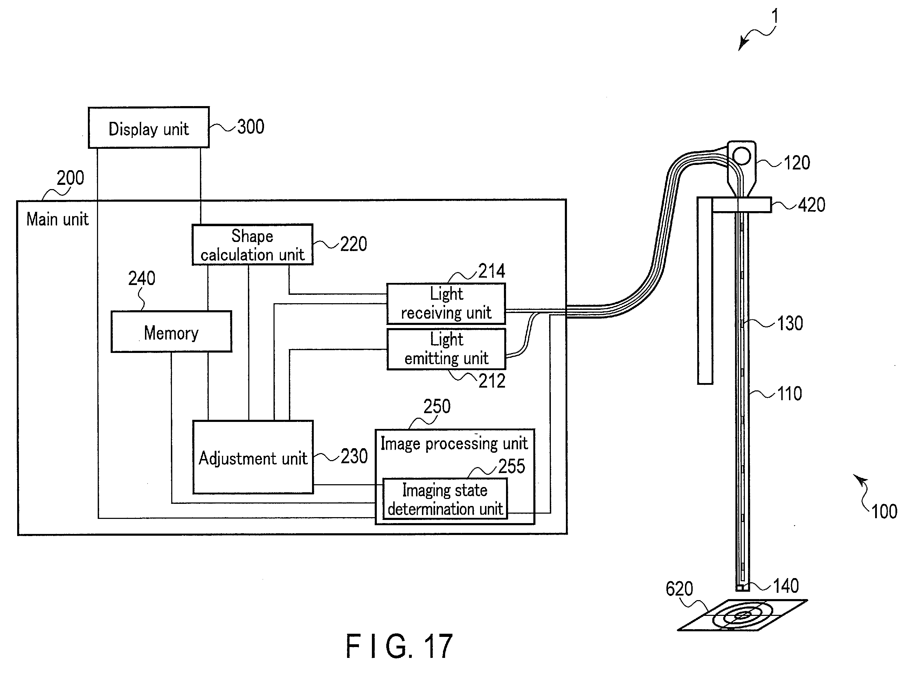

In a second example, a state determination pattern 620 is provided as shown in FIG. 17 so that the image sensor 140 functions as a holding state detection unit. This state determination pattern 620 has, for example, a predetermined geometrical design, and is provided at a position such that the geometrical design is photographed in the center of the angle of view of the image sensor 140 when the insertion device 100 is disposed in the insertion device holder 420 and the insertion portion 110 is vertically hanging.

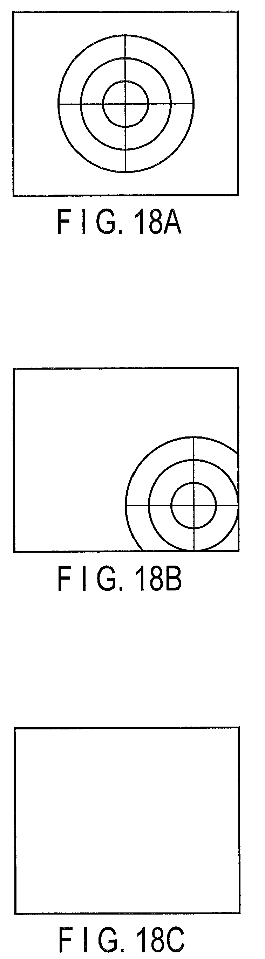

For example, an image in FIG. 18A is acquired by the image sensor 140 when the insertion device 100 is disposed in the insertion device holder 420 and the insertion portion 110 is vertically hanging. That is, in this case, the state determination pattern is imaged in the center. In contrast, for example, when the bending section 115 is bent, an image taken by the image sensor 140 is, for example, as shown in FIG. 18B. That is, in this case, the state determination pattern is imaged out of position. For example, when the insertion device 100 is not disposed in the insertion device holder 420, an image taken by the image sensor 140 is, for example, as shown in FIG. 18C. That is, the state determination pattern is not imaged.

An image of the state determination pattern 620 is stored in the memory 240. The image processing unit 250 includes an imaging state determination unit 255. The imaging state determination unit 255 compares the image taken by the image sensor 140 with the stored image (pattern matching), and on the basis of the result of the comparison, determines whether the insertion device 100 is disposed in the insertion device holder 420 and whether the insertion portion 110 is vertically hanging. The imaging state determination unit 255 outputs the determination result to the adjustment unit 230. The adjustment unit 230 determines an adjustment value when it is determined that the insertion device 100 is disposed in the insertion device holder 420 and the insertion portion 110 is vertically hanging.

According to this example, it is not necessary to additionally provide a sensor to detect the state of the insertion device 100, whether the insertion portion 110 is vertically hanging is easily determined, and an adjustment value can be precisely determined.

(Third Example)

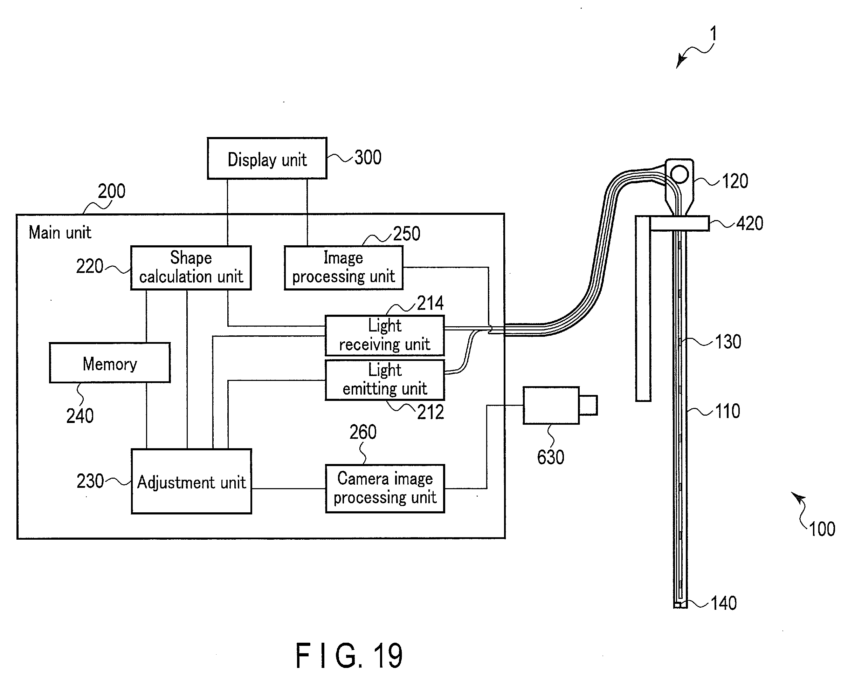

In this example, the insertion system 1 is provided with an external camera 630 as shown in FIG. 19. Moreover, the main unit 200 is provided with a camera image processing unit 260 which processes an image obtained by the external camera 630. The external camera 630 images the shape of the insertion portion 110. The external camera 630 outputs image data obtained by the imaging to the camera image processing unit 260. The camera image processing unit 260 analyzes the shape of the insertion portion 110 by, for example, pattern matching, and thereby determines whether the insertion device 100 is disposed in the insertion device holder 420 and whether the insertion portion 110 is vertically hanging. The camera image processing unit 260 outputs the determination result to the adjustment unit 230. The adjustment unit 230 starts the determination of an adjustment value on the basis of the determination result acquired from the camera image processing unit 260.

According to this example as well, whether the insertion portion 110 is vertically hanging is easily determined, and an adjustment value can be precisely determined.

A suitable combination of the first to third embodiments and their modifications above can be used. When a combination of the embodiments and the modifications are used, a more precise adjustment value is determined by the adjustment unit 230. As a result, the shape calculation unit 220 can precisely calculate the shape of the insertion portion 110.

**Claims**

1. An insertion system comprising:

   an insertion device which comprises an elongated flexible insertion portion;
   a shape sensor which detects a shape of the insertion portion;
   an insertion device holder which holds the insertion device at one end of the insertion portion so that the other end of the insertion portion hangs; and
   an adjustment unit which determines an adjustment value to adjust shape detection characteristics of the shape sensor when the insertion device is held by the insertion device holder.

2. The insertion system according to claim 1, further comprising a holding state detection unit which detects whether the insertion device holder is holding the insertion device,
   wherein the adjustment unit determines the adjustment value when the holding state detection unit detects that the insertion device is held by the insertion device holder.

3. The insertion system according to claim 2, wherein the holding state detection unit is disposed in the insertion device holder, and includes a switch a state of which changes in accordance with at least one of a mechanical change, an optical change, and an electric change.

4. The insertion system according to claim 3, wherein the state of the switch changes due to a weight of the insertion device held by the insertion device holder.

5. The insertion system according to any one of claims 1 to 4, further comprising an insertion portion state detection unit which acquires shape information regarding a shape of at least part of the insertion portion,
   wherein the adjustment unit determines the adjustment value based on the shape information.

6. The insertion system according to claim 5, wherein the shape information includes information regarding whether the shape of at least part of the insertion portion satisfies a predetermined condition, and

the adjustment unit determines the adjustment value when the shape of the insertion portion satisfies the predetermined condition.

7. The insertion system according to claim 5, wherein the insertion portion includes a bending section which actively bends,
   the insertion system further comprises an operation portion which operates a bending state of the bending section, and
   the insertion portion state detection unit acquires the shape information regarding a shape of the bending section based on an operation amount of the operation portion.

8. The insertion system according to claim 5, wherein the insertion portion state detection unit includes a gravity sensor provided in the insertion device, and the insertion portion state detection unit acquires the shape information based on a direction of a position where the gravity sensor is provided relative to a gravity direction.

9. The insertion system according to claim 5, wherein the insertion portion state detection unit includes a proximity sensor which detects whether the insertion portion exists in a position where the insertion portion is located when the insertion device is held by the insertion device holder.

10. The insertion system according to claim 5, wherein the insertion portion state detection unit includes proximity sensors which detect whether the insertion portion exists in positions where the insertion portion is located when the insertion device is held by the insertion device holder, the positions being different positions in a longitudinal direction of the insertion portion.

11. The insertion system according to claim 5, wherein the insertion device has an image sensor, and the insertion portion state detection unit includes an imaging state determination unit which acquires the shape information based on image information obtained from the image sensor.

12. The insertion system according to claim 5, wherein the insertion portion state detection unit includes an external camera configured to image the insertion portion when the insertion device is held by the insertion device holder, and
    a camera image processing unit which acquires the shape information based on an image taken by the external camera.

13. The insertion system according to claim 2, wherein the holding state detection unit further acquires shape information regarding a shape of at least part of the insertion portion, and
the adjustment unit determines the adjustment value based on the shape information when the holding state detection unit detects that the insertion device is held by the insertion device holder.

14. The insertion system according to claim 13, wherein the shape information includes information regarding whether the shape of at least part of the insertion portion satisfies a predetermined condition, and the adjustment unit determines the adjustment value when the shape of the insertion portion satisfies the predetermined condition.

15. The insertion system according to claim 13 or 14, wherein the holding state detection unit includes a proximity sensor configured to detect whether the insertion portion exists in a position where the insertion portion is located when the insertion device is held by the insertion device holder, determination of whether the insertion device is held by the insertion device holder and acquirement of the shape information being performed based on whether the insertion portion exists.

16. The insertion system according to claim 13 or 14, wherein the holding state detection unit includes proximity sensors configured to detect whether the insertion portion exists in positions where the insertion portion is located when the insertion device is held by the insertion device holder, the positions being different positions in a longitudinal direction of the insertion portion, determination of whether the insertion device is held by the insertion device holder and acquirement of the shape information being performed based on whether the insertion portion exists.

17. The insertion system according to claim 13 or 14, wherein
the insertion device includes an image sensor, and the insertion portion state detection unit includes an imaging state determination unit which determines whether the insertion device is held by the insertion device holder and which acquires the shape information based on image information obtained from the image sensor.

18. The insertion system according to claim 13 or 14, wherein the holding state detection unit includes an external camera configured to image the insertion portion when the insertion device is held by the insertion device holder, and a camera image processing unit which determines whether the insertion device is held by the insertion device holder and which acquires the shape information based on an image taken by the external camera.

19. The insertion system according to claim 2, wherein the holding state detection unit includes a proximity sensor configured to detect whether the insertion portion exists in a position where the insertion portion is located when the insertion device is held by the insertion device holder, determination of whether the insertion device is held by the insertion device holder being performed based on whether the insertion portion exists.

20. The insertion system according to claim 2, wherein the insertion device has an image sensor, and the holding state detection unit includes an imaging state determination unit which determines whether the insertion device is held by the insertion device holder based on image information obtained from the image sensor.

21. The insertion system according to claim 2, wherein the holding state detection unit includes an external camera configured to image the insertion portion when the insertion device is held by the insertion device holder, and a camera image processing unit which determines whether the insertion device is held by the insertion device holder based on an image taken by the external camera.

22. The insertion system according to claim 1, wherein the adjustment unit determines the adjustment value after waiting for a predetermined time after the insertion device has been held by the insertion device holder.

23. A method of adjusting shape detection characteristics of a shape sensor of an insertion system, the insertion system comprising an insertion device which includes an elongated flexible insertion portion and the shape sensor which detects a shape of the insertion portion, the method comprising:

detecting whether the insertion device is held at one end of the insertion portion so that the other end of the insertion portion hangs; and determining an adjustment value to adjust the shape detection characteristics of the shape sensor when the insertion device is held.

24. The method according to claim 23, wherein the insertion system further comprises an insertion device holder which holds the insertion device, the method further comprises determining whether the insertion device holder is holding the insertion device, and the determining the adjustment value is performed when the insertion device is determined to be held by the insertion device holder.

**25.** The method according to claim 24, further comprising acquiring shape information regarding a shape of at least part of the insertion portion,
wherein the determining the adjustment value is performed based on the shape information.

**26.** The method according to any one of claims 23 to 25, wherein the adjustment value is determined after a wait for a predetermined time after the insertion device has been held.

F I G. 1

FIG. 2

FIG. 3A

F I G. 3B

F I G. 3C

F I G. 4

120

125

500

420

110

# F I G. 5

Light receiving amount

912

914

Curvature
(curving amount)

−                    0                    +

# F I G. 6

Adjustment value
determination processing

S101 — Acquire holding state

S102

Insertion device is
held by insertion
device holder?

NO

YES

S103 — Compare current light receiving amount
with light receiving amount in initial state
in which insertion portion is straight

S104 — Adjust relation between
current light receiving amount and
light receiving amount in initial state
in which insertion portion is straight

End

F I G. 7

F I G. 8

F I G. 9

Display unit ~300

200

Main unit

100

Insertion device

~140

220~ Shape calculation unit

~250

Image processing unit

214

Image sensor

~135

Light receiving unit

~130

Shape detection portion

Memory

Light emitting unit

Shape sensor

240

212

Adjustment unit

Input device ~530

230

1

F I G. 10

Adjustment value
determination processing

S201 — Acquire holding state

S202

Insertion device is
held by insertion
device holder?                    NO

YES

S203 — Delay to start of adjustment

S204 — Compare current light receiving amount
with light receiving amount in initial state
in which insertion portion is straight

S205 — Adjust relation between
current light receiving amount and
light receiving amount in initial state
in which insertion portion is straight

End

F I G. 11

F I G. 12

EP 3 031 385 A1

Adjustment value
determination processing

S301 — Acquire holding state

S302 — Calculate curving amount
based on operation amount

S303

Insertion device is
held by insertion
device holder?  — NO

YES

S304

Shape of curving portion
is straight?  — NO

YES

S305 — Compare current light receiving amount
with light receiving amount in initial state
in which insertion portion is straight

S306 — Adjust relation between
current light receiving amount and
light receiving amount in initial state
in which insertion portion is straight

End

F I G. 13

F I G. 14

EP 3 031 385 A1

Adjustment value
determination processing

S401 — Acquire holding state

S402 — Calculate curving amount
based on operation amount

S403

Insertion device is
held by insertion
device holder?　　NO

YES

S404 — Compare current light
receiving amount with detected light
receiving amount in initial state
in which insertion portion is curved

S405 — Adjust relation between
current light receiving amount and
light receiving amount in initial state

End

F I G. 15

F I G. 16

EP 3 031 385 A1

F I G. 17

F I G. 18A

F I G. 18B

F I G. 18C

F I G. 19

EP 3 031 385 A1

## INTERNATIONAL SEARCH REPORT

| | International application No. |
|---|---|
| | PCT/JP2014/067351 |

A. CLASSIFICATION OF SUBJECT MATTER
*A61B1/00*(2006.01)i, *A61B1/04*(2006.01)i, *A61B5/06*(2006.01)n

According to International Patent Classification (IPC) or to both national classification and IPC

B. FIELDS SEARCHED

Minimum documentation searched (classification system followed by classification symbols)
A61B1/00-1/32, A61B5/06

Documentation searched other than minimum documentation to the extent that such documents are included in the fields searched
| Jitsuyo Shinan Koho | 1922–1996 | Jitsuyo Shinan Toroku Koho | 1996–2014 |
| Kokai Jitsuyo Shinan Koho | 1971–2014 | Toroku Jitsuyo Shinan Koho | 1994–2014 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2009-516574 A (NeoGuide Systems, Inc.), 23 April 2009 (23.04.2009), paragraph [0057] & US 2007/0161857 A1 & WO 2007/062179 A2 | 1-26 |
| A | JP 2013-106752 A (National Cancer Center), 06 June 2013 (06.06.2013), fig. 26 (Family: none) | 1-26 |
| A | JP 2007-044412 A (Pentax Corp.), 22 February 2007 (22.02.2007), entire text; all drawings (Family: none) | 1-26 |

☒ Further documents are listed in the continuation of Box C.　　☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 15 August, 2014 (15.08.14) | 02 September, 2014 (02.09.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2014/067351

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2011-030735 A  (Fujifilm Corp.),<br>17 February 2011 (17.02.2011),<br>entire text; all drawings<br>(Family: none) | 1-26 |
| A | JP 2007-163462 A  (Biosense Webster, Inc.),<br>28 June 2007 (28.06.2007),<br>fig. 3<br>& US 2007/0085528 A1    & EP 1776923 A1<br>& KR 10-2007-0042872 A | 1-26 |

Form PCT/ISA/210 (continuation of second sheet) (July 2009)

**EP 3 031 385 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003052614 A